# EUROPÄISCHE PATENTSCHRIFT

(11) **EP 3 036 522 B1**
(45) Veröffentlichungstag und Bekanntmachung des Hinweises auf die Patenterteilung: **02.10.2019**
(21) Anmeldenummer: 14739382.1
(22) Anmeldetag: 08.07.2014
(51) Int. Cl.: G01N 19/04, G01N 33/32

(54) **PRÜFUNG DER HAFTUNG VON ELASTISCHEN KLEBSTOFFEN ODER ELASTISCHEN DICHTUNGSMATERIALIEN AUF OBERFLÄCHEN VON BAUTEILEN**
TESTING THE ADHERENCE OF ELASTIC ADHESIVES OR ELASTIC SEALING MATERIALS ON SURFACES OF COMPONENTS
CONTRÔLE DE L'ADHÉRENCE D'ADHÉSIFS ÉLASTIQUES OU DE MATÉRIAUX D'ÉTANCHÉITÉ ÉLASTIQUES SUR DES SURFACES DE PIÈCES

(30) Priorität: 22.08.2013 DE 102013216710
(43) Veröffentlichungstag der Anmeldung: 29.06.2016
(73) Patentinhaber: Bombardier Transportation GmbH, 10785 Berlin (DE)
(72) Erfinder: TELLO, Waissi, 34292 Ahnatal (DE); KLEINÖDER, Helmuth, 34132 Kassel (DE)
(74) Vertreter: Patentanwälte Bressel und Partner mbB
(86) Internationale Anmeldenummer: PCT/EP2014/064607
(87) Internationale Veröffentlichungsnummer: WO 2015/024700

(56) Entgegenhaltungen:
- EP-A2- 2 431 728
- FR-A1- 2 572 216
- US-A1- 2003 054 740

## Beschreibung

Die Erfindung betrifft ein Verfahren zur Prüfung der Haftung von elastischen Klebstoffen oder elastischen Dichtungsmaterialien auf Oberflächen von Bauteilen. Die Erfindung betrifft ferner eine entsprechende Anordnung zur Prüfung der Haftung.

Insbesondere im Schienenfahrzeugbau werden verschiedene Bauteile zunehmend durch Klebstoffverbindungen zusammengefügt. Außerdem kommen ebenfalls insbesondere im Schienenfahrzeugbau zunehmend Dichtungen vor, die durch elastische Dichtungsmaterialien gebildet werden, welche auf die Oberfläche von einem Bauteil aufgebracht werden und durch Adhäsion an der Oberfläche haften. Bei den Dichtungsmaterialien handelt es sich daher ebenfalls um Klebstoffe, die jedoch nicht zum Herstellen einer Fügeverbindung zweier Fügeteile (Bauteile) verwendet werden. Wenn in dieser Beschreibung daher von Klebstoffen die Rede ist und lediglich die Haftung des Klebstoffs an der Oberfläche eines einzigen Bauteils betrachtet wird, ist dadurch analog auch der Fall eines Dichtungsmaterials erfasst. Außer in den beigefügten Patentansprüchen und einzelnen Teilpassagen der Beschreibung umfasst der Begriff Klebstoff daher auch Dichtungsmaterialien.

In Abhängigkeit von den Klebstoffeigenschaften bei Applikation (Viskosität, Oberflächenspannung), von der Bauteiloberfläche (Rauigkeit, Oberflächenspannung) und/oder einer geforderten Langzeitbeständigkeit werden ggf. Stoffe vor der Applikation des Klebstoffes auf die Bauteiloberfläche aufgetragen, um die Haftung des Klebstoffes auf der Bauteiloberfläche zu verbessern. Dabei handelt es sich insbesondere um sogenannte Aktivatoren und/oder Primer. Aktivatoren sind insbesondere Reinigungslösungen zur Vorbehandlung der Bauteiloberflächen. Die Bauteiloberflächen werden z. B. mit einem sauberen, fusselfreien und mit Aktivator getränkten Tuch in einem Arbeitsgang dünn aufgetragen. Zur Haftverbesserung kann es auch erforderlich sein, einen Voranstrich auf die Bauteiloberfläche aufzutragen. Bei diesem Voranstrich handelt es sich um die sogenannten Primer. Die Verarbeitung des Primers erfolgt in der Regel mit Pinsel, Melaminharzschäumen, Filzflasche oder anderen Applikationsgeräten. Der Primer wird gleichmäßig dünn, aber deckend aufgetragen.

Bei den sogenannten Aktivatoren oder Primern handelt es sich um klebstoffhaftvermittelnde Stoffe. Wenn im Folgenden von Haftvermittlern die Rede ist, sind damit insbesondere die Aktivatoren und/oder Primer gemeint.

Die Erfindung betrifft insbesondere das Dickschichtkleben mit elastischen Klebstoffen. Darunter werden Klebverbindungen verstanden, deren Klebspaltdicke größer als 1,5 mm ist. Ferner betrifft die Erfindung insbesondere Klebstoffe, die in einem abgebundenen Zustand gummielastische Eigenschaften besitzen und vorzugsweise Schub-(Wechsel-) Verformungen von mehr als 15 % der Klebspaltdicke auf Dauer schädigungsfrei ertragen. Solche Klebstoffverbindungen und Klebstoffe sind insbesondere auch Gegenstand des Merkblatts 1618 (Januar 2002) des Deutschen Verbandes für Schweißen und verwandte Verfahren e.V. In Anlage 3 dieses Merkblatts wird zur Prüfung des Klebstoffs und der Klebverbindung ein sogenannter Peeltest/Raupentest beschrieben. Zur Qualitätssicherung und/oder Prüfung der Langzeitbeständigkeit unter spezifischen Alterungszyklen (z.B. gegen Feuchtigkeitseinwirkung wie in dem Merkblatt beschrieben) wird die Haftung des Klebstoffs auf einer Bauteiloberfläche geprüft und beurteilt. Die Prüfvorschrift betrifft explizit auch Dichtungsmaterialien. Für die Prüfung wird eine Rundraupe von 10 mm Durchmesser und einer Länge von mindestens 50 mm auf die Bauteiloberfläche aufgetragen. Auch bei Nachweisführung der Langzeitbeständigkeit werden mindestens 50 mm pro Lagerbedingung und Prüfung appliziert. Nach dem Abbinden des Klebstoffs wird die Klebstoffraupe unter einem Winkel von 130° bis 160° gegen die Bauteiloberfläche in Längsrichtung der Raupe mit einer Spitzzange von der Bauteiloberfläche abgezogen.

Wenn sich während des Abziehens innerhalb der Klebstoffraupe Brüche des Klebstoffmaterials bilden (sogenannte Kohäsionsbrüche), kann dies zum Abreißen desjenigen Teils der Klebstoffraupe führen, in den mit der Spitzzange die Schälkraft (d.h. die den Klebstoff von der Bauteiloberfläche abziehende Kraft, d.h. Ablösekraft) eingeleitet wird. Ein fortschreitender Kohäsionsbruch führt in jedem Fall dazu, dass die mit der Spitzzange eingeleitete Schälkraft (Ablösekraft) zunehmend schwächer auf die Grenzfläche zwischen Klebstoffraupe und Bauteiloberfläche einwirkt. Da jedoch die Haftung der Klebstoffraupe an der Bauteiloberfläche nicht lediglich an einer Stelle der Grenzfläche geprüft und bewertet werden soll, sondern mindestens über die genannte Länge von 50 mm, wird mit einem scharfen spitzen Messer schräg zur Oberflächennormale des Bauteils verlaufend bis in das Material des Bauteils eingeschnitten und auf diese Weise der Vorgang des Abschälens der Klebstoffraupe unterstützt. Das Einschneiden bis in das Material des Bauteils hinein wird in Längsrichtung der Klebstoffraupe etwa alle 5 bis 10 mm wiederholt. Für die aufeinanderfolgend durchgeführten Einschnitte mit dem scharfen spitzen Messer soll ein Zeitabstand von 3 s eingehalten werden, während dem das Klebstoffmaterial andauernd durch Einleiten der Schälkraft belastet wird. Das Einschneiden bis in das Material des Bauteils hinein findet insbesondere bei lackierten oder grundierten Bauteiloberflächen statt. In diesem Fall wird mit dem scharfen spitzen Messer bis in den Trägeruntergrund eingeschnitten, der sich unterhalb der Lackschicht bzw. Grundierungsschicht befindet. Der Schichtaufbau an der Bauteiloberfläche kann aus einer Schicht oder aus mehreren Teilschichten bestehen.

Ein ähnliches Verfahren zur Prüfung der Klebstoffhaftung einer Klebstoffraupe wird in der deutschen Industrienorm (DIN) 54457 (September 2007), herausgegeben durch das Deutsche Institut für Normung e.V., Berlin, beschrieben.

Nach dem Abschälen der Klebstoffraupe wird das sogenannte Bruchbild ausgewertet, insbesondere nach DIN EN ISO 10365. Je größer der durch Kohäsionsbrüche bewirkte Anteil der Ablösung der Klebstoffraupe von der Bauteiloberfläche im Verhältnis zu dem Lösen der Adhäsion zwischen Klebstoffraupe und Bauteiloberfläche ist, desto besser ist die Haftung des Klebstoffs an der Bauteiloberfläche.

Bei lackierten bzw. grundierten Bauteilen wird mit dem scharfen spitzen Messer bis in den Trägeruntergrund eingeschnitten, der sich unterhalb der Lackschicht bzw. Grundierungsschicht befindet. Dies führt zu einer lokalen Beschädigung des Lackaufbaus und kann z. B. zu Korrosionserscheinungen führen, wenn der lokal beschädigte Bereich nicht neu lackiert bzw. grundiert wird. Eine derartige sichtbare lokale Beschädigung wird von Kunden und Anwendern des gefertigten Gegenstandes als Mangel angesehen.

Die Beschädigung der Bauteiloberfläche ist auch bei Bauteilen wesentlich, die Faseranteile aufweisen, z.B. glasfaserverstärkte Kunststoffe (GFK). Bei solchen Bauteilen wird bis in Fasern eingeschnitten, wodurch es zum Ausreißen von Fasern beim Abschälen der Klebstoffraupe kommen kann und die Fasern schließlich freiliegen und somit nicht mehr ausreichend gegen Feuchtigkeitseinwirkung geschützt sind. Zudem bleibt die Beschädigung sichtbar bestehen, wenn das Bauteil für die eigentliche Fertigung wieder verwendet wird. Eine derartige lokale Beschädigung wird von Kunden und Anwendern des gefertigten Gegenstandes als Mangel angesehen.

Ein anderer Gegenstand, der insbesondere im Schienenfahrzeugbau mit anderen Bauteilen verklebt wird, ist eine Fensterscheibe. Am Rand von Scheiben befindet sich üblicherweise ein undurchsichtiger Farbauftrag, der meist durch Drucken auf den Rand der Scheibe aufgetragen wird. Typischerweise handelt es sich dabei um einen Keramiksiebdruck. Der Farbauftrag wird bei dem zuvor beschriebenen Prüfverfahren durch das Einschneiden mit dem scharfen spitzen Gegenstand beschädigt, wobei die Beschädigung sichtbar bestehen bleibt, wenn das Bauteil für die eigentliche Fertigung wieder verwendet wird. Eine solche sichtbare lokale Beschädigung wird von Kunden und Anwendern des gefertigten Gegenstandes als Mangel angesehen.

Nachteilig an dem zuvor beschriebenen Prüfverfahren ist die Beschädigung des Bauteils mit dem scharfen spitzen Messer. Zur Vermeidung von Beschädigungen an Bauteilen, die in der Fertigung industriell oder handwerklich hergestellter Gegenstände verwendet werden, sind daher zusätzliche Muster verwendet worden. Die Muster sollen dieselben Eigenschaften aufweisen wie das zu klebende Bauteil, jedenfalls in Bezug auf den Bereich der Bauteiloberfläche und seine Wechselwirkung mit dem Klebstoff. Der oben beschriebene Schältest kann dann lediglich an dem Muster durchgeführt werden.

Dies hat jedoch den Nachteil, dass die Eigenschaften des Musters von den Eigenschaften des Bauteils abweichen können, zumal eine Herstellung des Musters unter gleichen Bedingungen wie bei der Fertigung des Bauteils nicht stets sichergestellt werden kann Ferner hat dies den Nachteil, dass zusätzliche Bauteile als Muster beschafft werden müssen. Werden die Muster nicht in exakt gleicher Weise wie die in dem Fertigungsprozess zu verwendenden Bauteile behandelt, sind Unterschiede in den Eigenschaften zu erwarten und entsteht zusätzlicher Fertigungsaufwand, z.B. Raumbedarf für den Ort, an dem das Muster mit Klebstoff versehen wird. Um die Herstellung des Musters begleitend zur Herstellung des Bauteils und unter möglichst gleichen Bedingungen zu gewährleisten, ist ebenfalls höherer Aufwand erforderlich.

FR 2 572 216 A1 offenbart ein Verfahren zur Kontrolle der Haftung einer auf einer Oberfläche aufgebrachten Schicht durch Abziehen dieser Schicht. Vor dem Aufbringen der Schicht wird in einem Randbereich eine weitere Schicht mit geringerer Haftung aufgebracht. Das Abziehen der zu untersuchenden Schicht beginnt in dem Randbereich mit geringerer Haftung, indem dieser Bereich in eine Vorrichtung zum Messen der Haftung eingespannt wird. Als Anwendungsbereich wird allgemein auf Schichten oberhalb von anderen Schichten und speziell auf die Kontrolle der Haftung von metallischen Schichten auf Halbleitersubstraten verwiesen.

Es ist eine Aufgabe der vorliegenden Erfindung, den Aufwand für die Prüfung der Haftung von Klebstoffen und Dichtungsmaterialien auf Bauteiloberflächen zu verringern.

Der Schutzbereich ergibt sich aus den beigefügten Patentansprüchen. Anspruch 1 ist auf ein Verfahren zur Prüfung der Haftung von elastischen Klebstoffen oder elastischen Dichtungsmaterialien auf Oberflächen von Bauteilen gerichtet. Anspruch 7 ist auf eine Anordnung zur Prüfung der Haftung von elastischen Klebstoffen oder elastischen Dichtungsmaterialien auf Oberflächen von Bauteilen gerichtet.

Insbesondere sollen die oben genannten Nachteile des beschriebenen bekannten Prüfungsverfahrens vermieden werden.

Gemäß einer Grundidee der vorliegenden Erfindung wird zur Vorbereitung der Prüfung der Haftung von elastischen Klebstoffen oder elastischen Dichtungsmaterialien auf Bauteiloberflächen zumindest ein Teilbereich der Bauteiloberfläche mit einem Material versehen, das sich nach dem Aufbringen des Klebstoffs auf die Bauteiloberfläche zwischen dem Klebstoff und der Bauteiloberfläche befindet. Unter der Formulierung "zumindest ein Teilbereich der Bauteiloberfläche" wird verstanden, dass auch mehr als ein Teilbereich der Bauteiloberfläche mit dem zusätzlichen Material versehen werden kann. Dies wird auch bevorzugt. In jedem Fall befindet sich der Klebstoff nach dem Aufbringen auf der Bauteiloberfläche sowohl auf dem zumindest einen (ersten) Teilbereich, der mit dem zusätzlichen Material versehen ist, und außerdem auf zumindest einem (zweiten) Teilbereich der Bauteiloberfläche, in dem der Klebstoff in unmittelbarem Kontakt mit der Bauteiloberfläche ist. Während der Klebstoff daher in dem ersten Teilbereich oder den ersten Teilbereichen nicht unmittelbar in Kontakt mit der Bauteiloberfläche ist und daher auch nicht unmittelbar an der Bauteiloberfläche haften kann, hat der Klebstoff in dem zumindest einen zweiten Teilbereich den für die Prüfung der Haftung erforderlichen unmittelbaren Kontakt. Wenn sich der Klebstoff nun durchgehend über die Grenze zwischen einem ersten und einem zweiten Teilbereich hinwegerstreckt, übt das zusätzliche Material auf dem ersten Teilbereich die Funktion des scharfen spitzen Messers aus dem oben beschriebenen bekannten Prüfverfahren aus. Dies gilt insbesondere dann, wenn das zusätzliche Material so gewählt ist, dass der Klebstoff auf der Oberfläche des zusätzlichen Materials schlechter haftet als unmittelbar auf der Oberfläche des Bauteils, oder wenn das zusätzliche Material schlechter auf der Bauteiloberfläche haftet als der Klebstoff auf dem zusätzlichen Material. Unter einer schlechteren Haftung wird auch der Fall verstanden, dass der Klebstoff bzw. das Material gar nicht haftet. Dies gilt aber auch dann, wenn beim Aufbringen einer Schälkraft auf den Klebstoff (insbesondere die Klebstoffraupe), die ablösend in dem ersten Teilbereich wirkt, das zusätzliche Material bei geringerer Schälkraft von der Bauteiloberfläche abgelöst wird als für die Ablösung des Klebstoffs von der Bauteiloberfläche in dem benachbarten zweiten Oberflächenbereich erforderlich ist. Im Ergebnis kann das zusätzliche Material daher in Bezug auf die Haftung des Klebstoffs auf der Bauteiloberfläche als Antihaft-Material bezeichnet werden.

Insbesondere wird das zusätzliche Material als Antihaft-Materialschicht auf den zumindest einen ersten Teilbereich der Bauteiloberfläche aufgebracht. Dabei ist die Schichtdicke insbesondere so klein gewählt, dass eine gute Benetzung des Klebstoffes auf dem zumindest einen zweiten Teilbereichen ermöglicht wird und vorzugsweise kleiner als ein Fünftel und vorzugsweise kleiner als ein Zehntel der Dicke des zur Prüfung der Haftung aufgetragenen Klebstoffs. Das Auftragen als Antihaft-Materialschicht hat den Vorteil, dass der Verlauf der Grenzfläche zwischen dem auf das zusätzliche Material aufgebrachten Klebstoffs dem Verlauf der Bauteiloberfläche folgt und der Vorgang der Ablösung des Klebstoffs zum Zweck der Haftungsprüfung in gleicher Weise wie bei dem bekannten Verfahren ausgeführt werden kann.

Die Verwendung eines Antihaft-Materials in dem zumindest einen ersten Teilbereich hat aber in jedem Fall den oben bereits angedeuteten Vorteil, dass ohne die Verwendung eines scharfen spitzen Gegenstandes die Ablösung des Klebstoffs in dem ersten Teilbereich erleichtert ist und dadurch trotz oder vor dem Auftreten eines Kohäsionsbruchs in dem Klebstoff eine Schälkraft ausgeübt werden kann, die in einem an den ersten Teilbereich angrenzenden zweiten Teilbereich der Bauteiloberfläche ablösend auf den Klebstoff wirkt. Beschädigungen der Bauteiloberfläche durch einen scharfen spitzen Gegenstand werden daher vermieden. Ansonsten kann der Prüfvorgang insbesondere genauso wie oben oder in den oben zitierten Normen und dem oben zitierten technischen Merkblatt beschrieben durchgeführt werden.

Insbesondere kann sich der auf die verschiedenen Teilbereiche der Bauteiloberfläche aufgebrachte Klebstoff durchgehend über zumindest eine Grenze zwischen einem ersten Teilbereich und einem zweiten Teilbereich der Bauteiloberfläche hinwegerstrecken, z.B. in einer Längsrichtung, etwa in Form einer sich in der Längsrichtung erstreckenden Klebstoffraupe. Bei der Längsrichtung handelt es sich insbesondere um eine gerade Richtung. Die auf den Klebstoff ausgeübte Schälkraft, die für den Klebstoff von der Oberfläche ablösend wirkt, wird insbesondere (wie auch bei dem bekannten Prüfverfahren) so ausgeübt, dass eine Komponente der Schälkraft in der Längsrichtung wirkt und eine andere Komponente der Schälkraft ablösend senkrecht zur Oberfläche des Bauteils wirkt. Wie erwähnt erstreckt sich dabei die Längsrichtung über eine Grenze oder mehrere Grenzen zwischen jeweils einem ersten und einem zweiten Teilbereich der Bauteiloberfläche hinweg. Vorzugsweise erstreckt sich die Längsrichtung über zumindest einen ersten Teilbereich hinweg, der an seinen einander gegenüberliegenden Rändern jeweils an einen zweiten Teilbereich der Bauteiloberfläche angrenzt.

Unter dem Aushärten des Klebstoffs wird insbesondere das oben erwähnte Abbinden verstanden. Elastische Klebstoffe werden insbesondere für den Fall des Dickschichtklebens typischerweise in hochviskosem Zustand auf die Bauteiloberfläche aufgebracht. In der Regel verfügen einkomponentige, feuchtigkeitshärtende Polyurethanklebstoffe, silan-terminierte Polyether, silanmodifzierte Polyurethane, beschleunigte Polyurethansysteme (sogenannte "boosterfähige" Klebstoffe) oder Silikonsysteme über die benötigten elastischen Eigenschaften für die Raupenschälprüfung. Jedoch beschränkt sich die Erfindung nicht auf die genannten Klebstoffsysteme, sondern verweist lediglich auf die benötigten elastischen Eigenschaften nach der Abbindung des Klebstoffes bzw. Dichtungsmaterials, wie dies bereits im bekannten Prüfverfahren definiert ist. Um das Abbinden zu ermöglichen, kann es bei bestimmten Ausgestaltungen von Klebstoffen auch erforderlich sein zwei Komponenten miteinander zu vermischen.

Der Klebstoff wird insbesondere in der gleichen Weise auf die Bauteiloberfläche aufgebracht wie bei den bisher bekannten Prüfverfahren. Z.B. kann der Klebstoff in einer Kartusche unter Druck gesetzt werden und aus einer Öffnung der Kartusche austreten. Alternativ kann der Klebstoff mittels einer Dosieranlage mit Pumpentechnik (z.B. Schöpfkolbenpumpe und/oder Zahnradpumpe) in den Bereich an der Bauteiloberfläche gefördert werden. Der austretende Klebstoff wird auf die gewünschten Bereiche der Bauteiloberfläche aufgebracht. Es können auch weitere Hilfsmittel wie z.B. Spatel zum Aufbringen auf die Bauteiloberfläche verwendet werden.

Je nach Bauteiloberfläche, Langzeitbeanspruchung und elastischem Klebstoff oder Dichtungsmaterial werden für eine gute Haftung eventuell haftvermittelnde Stoffe benötigt, sogenannte Aktivatoren und/oder Primer, die ggf. nach der Reinigung als Haftvermittlung für den elastischen Klebstoff oder Dichtungsmaterial zur Bauteiloberfläche aufgetragen werden. Dies ist an sich bereits bekannt. Insbesondere findet daher vor dem Aufbringen des Klebstoffs in dem ersten Teilbereich der Bauteiloberfläche und/oder in dem zweiten Teilbereich der Bauteiloberfläche optional statt:
i) eine Reinigung und/oder mechanische Oberflächenvorbereitung (z.B. Schleifen oder Strahlen bei metallischen Oberflächen) der Bauteiloberfläche und/oder
ii) eine Aufbringung zumindest eines haftvermittelnden Stoffes auf die Bauteiloberfläche.

Die Antihaft-Materialschicht kann in dem zumindest einen ersten Teilbereich vor, während oder nach dem Schritt i) und/oder vor, während oder nach dem Schritt ii) auf die Bauteiloberfläche aufgebracht werden. Vorzugsweise wird dabei ein Antihaft-Material verwendet, das nach Schritt c) eine rückstandsfreie Ablösung von der Bauteiloberfläche ermöglicht. Auf Bauteiloberflächen, die eine Prüfung der Haftung außerhalb der unmittelbaren Klebfläche ermöglichen, wird vorzugsweise nach Schritt i) und/oder nach Schritt ii) die Antihaft- Materialschicht aufgebracht.

Die Schälkraft auf den ausgehärteten Klebstoff wird vorzugsweise wie bei dem bekannten Prüfungsverfahren mit einer Spitzzange auf den Klebstoff aufgebracht. Insbesondere hat der ausgehärtete Klebstoff die Form einer Klebstoffraupe.

In dem oben beschriebenen bekannten Prüfverfahren kommt es vor, dass die ausgeübte Schälkraft nicht ausreicht, um den Klebstoff, insbesondere die Klebstoffraupe, weiter abzuschälen, so dass hier erneut mit einem scharfen, spitzen Gegenstand bis auf die Haftfläche eingeschnitten werden muss, um die Klebstoffraupe weiter abzuschälen. Die Erfindung schafft Abhilfe, da an einem ersten Teilbereich der Bauteiloberfläche, insbesondere durch erneutes Ansetzen eines Werkzeugs (z. B. einer Spitzzange), eine Schälkraft auf den Klebstoff ausgeübt werden kann, die für eine Ablösung ausreicht. Die auf den Klebstoff ausgeübte Schälkraft führt in dem ersten Teilbereich wegen der reduzierten Haftung zu einer erzwungenen Ablösung des Klebstoffes vom Antihaft-Material und/oder zu einer Ablösung des Antihaft- Materials von der Bauteiloberfläche. Mit fortschreitender Ablösung des Klebstoffes von dem ersten Teilbereich wirkt die Schälkraft auch in einem benachbarten zweiten Teilbereich.

Insbesondere beruht eine Ausführungsform der Erfindung auf einer vordefinierten Länge eines ersten Teilbereichs mit Antihaft-Materialschicht zwischen zwei zweiten Teilbereichen ohne Antihaft-Materialschicht. Durch diesen Abstand der zweiten Teilbereiche zueinander wird sichergestellt, dass die Schälkraft - trotz Verzicht auf einen Einschnitt des Klebstoffes mittels eines scharfen, spitzen Gegenstands - in ansonsten gleicher Weise wie bei dem bekannten Prüfverfahren auf den Klebstoff ausgeübt werden kann. Dabei kann die Schälkraft nacheinander an den zweiten Teilbereichen auf den Klebstoff ausgeübt werden. Zum Beispiel wird die Schälkraft zunächst auf den Klebstoff an einem der zweiten Teilbereiche ausgeübt und kann auf diese Weise festgestellt werden, inwieweit der Klebstoff an diesem zweiten Teilbereich ablösend wirkt. Sollte mit fortschreitender Ablösung des Klebstoffes von diesem zweiten Teilbereich die ausgeübte Schälkraft nicht mehr ausreichen, um den Klebstoff, insbesondere die Klebstoffraupe, weiter abzuschälen, kann die Schälkraft nun auf den Klebstoff an dem ersten Teilbereich ausgeübt werden und mit fortschreitender Ablösung des Klebstoffes von dem ersten Teilbereich auch auf den anderen zweiten Teilbereich ausgeübt werden.

Insbesondere kann der Klebstoff oder das Dichtungsmaterial daher derart auf die Bauteiloberfläche aufgebracht werden, dass sich der Klebstoff oder das Dichtungsmaterial in einer Längsrichtung zumindest von einem der zweiten Teilbereiche über einen der ersten Teilbereiche zu einem der zweiten Teilbereiche erstreckt. Insbesondere erstreckt sich der Klebstoff oder das Dichtungsmaterial in der Längsrichtung zumindest von einem der zweiten Teilbereiche über einen der ersten Teilbereiche zu einem anderen der zweiten Teilbereiche. Die beiden zweiten Teilbereiche können jedoch auch miteinander verbunden sein, etwa über einen Abschnitt, der sich parallel zur Längsrichtung erstreckt.

Wie auch bei dem bekannten Prüfungsverfahren, z.B. im Fall einer Klebstoffraupe, kann die Schälkraft eine Kraftkomponente aufweisen, die in der Längsrichtung des Klebstoffs wirkt.

Zu Beginn der Aufbringung einer Schälkraft auf den Klebstoff wird die Schälkraft vorzugsweise dort in den Klebstoff eingeleitet, wo sich ein erster Teilbereich befindet. Dadurch wird die anschließende Aufbringung einer Schälkraft auf den Klebstoff in einem benachbarten zweiten Teilbereich erleichtert. Es wird auch zu Beginn des Schälvorganges kein Schnitt in den Klebstoff bis in den Untergrund benötigt, um das Abschälen des Klebstoffes zu ermöglichen.

Durch die Aufbringung der Schälkraft auf den Klebstoff an dem genannten zweiten Teilbereich wird eine Kraft auf den Klebstoff ausgeübt, die je nach Stärke der Haftung des Klebstoffs von dem zweiten Teilbereich ablösend wirken kann. Es findet somit eine Prüfung der Haftung des Klebstoffs in diesem zweiten Teilbereich statt. Insbesondere wie in dem bekannten Prüfungsverfahren wird die Stärke der Schälkraft kontinuierlich und/oder stufenartig erhöht, bis innerhalb des Klebstoffs ein Kohäsionsbruch und/oder eine Ablösung des Klebstoffs von dem zweiten Teilbereich auftritt. Auf diese Weise kann die Haftung des Klebstoffs an dem zweiten Teilbereich beurteilt werden. Eine etwaig auftretende Ablösung des Klebstoffs tritt dabei in der Längsrichtung auf. Wenn die Ablösung die Grenze zwischen dem zweiten Teilbereich und dem ersten Teilbereich erreicht, schreitet bei weiter wirkender Schälkraft die Ablösung fort und löst den Klebstoff auch von dem ersten Teilbereich ab. Der Grund dafür liegt in der schlechteren Haftung des Klebstoffs an dem ersten Teilbereich und/oder an der nicht vorhandenen oder schlechten Haftung der Antihaft-Materialschicht an dem ersten Teilbereich der Bauteiloberfläche. Sollte ein Kohäsionsbruch innerhalb des Klebstoffs auftreten und soweit fortschreiten, dass die ablösend wirkende Kraft geringer wird, kann die Zugkraft an anderer Stelle auf den Klebstoff aufgebracht werden, insbesondere nahe der Grenze zwischen dem zweiten Teilbereich und dem ersten Teilbereich oder an dem ersten Teilbereich. Daher ist es auf einfache Weise möglich, nach der Ablösung oder der versuchten Ablösung des Klebstoffs von dem zweiten Teilbereich und ohne Einsatz eines scharfen Werkzeugs, das den Klebstoff von der Bauteiloberfläche abschält, eine potentiell ablösende Schälkraft auch so in den Klebstoff einzuleiten, dass sie in dem aus Sicht des ersten Teilbereichs in der Längsrichtung gegenüberliegenden zweiten Teilbereich wirkt.

In bevorzugter Ausgestaltung wird/ist der Klebstoff oder das Dichtungsmaterial derart auf die Bauteiloberfläche aufgebracht, dass sich der Klebstoff oder das Dichtungsmaterial in der Längsrichtung über sich abwechselnd hintereinander angeordnete erste und zweite Teilbereiche der Bauteiloberfläche erstreckt.

Bei dieser Ausgestaltung kann insbesondere der zuvor beschriebene Prozess des Einbringens einer Schälkraft in den Klebstoff über mehrere zweite Teilbereiche hinweg ausgeführt werden. Dadurch kann wie bei den bekannten Prüfverfahren die Haftung des Klebstoffs auf der Bauteiloberfläche über eine ausreichend große Länge geprüft werden.

Vorzugsweise sind die durch die Abmessungen der zweiten Teilbereiche in der Längsrichtung definierten Abstände der ersten Teilbereiche gleich groß. Dadurch werden für die verschiedenen zweiten Teilbereiche gleiche Voraussetzungen zur Prüfung der Haftung des Klebstoffs geschaffen. Es kann daher zuverlässig beurteilt werden, ob der Klebstoff an den verschiedenen zweiten Teilbereichen der Bauteiloberfläche gleich gut oder unterschiedlich haftet.

Nach der Durchführung des Prüfverfahrens kann (optional nach Aufbringung zumindest eines haftvermittelnden Stoffes auf die Bauteiloberfläche) erneut Klebstoff auf dieselben Teilbereiche (einschließlich des zumindest einen zweiten Teilbereichs, jedoch ohne die Antihaft-Materialschicht) aufgebracht werden und dadurch während der Fertigung eines Gegenstandes eine Klebstoffverbindung oder eine Dichtung hergestellt werden.

Bei einem Kohäsionsbruchanteil mit einem vorgegebenen Mindest-Prozentsatz (z. B. von 75%) auf der geprüften Oberfläche, die durch die zweiten Teilbereiche gebildet wird, handelt es sich um eine gute bzw. ausreichende Haftung wie auch in dem bekannten Prüfverfahren. Eine solche erfolgreich geprüfte Bauteiloberfläche ermöglicht es, das Bauteil für die eigentliche Fertigung wieder bzw. weiter zu verwenden. Ein Kohäsionsbruchanteil mit einem bestimmten Prozentsatz bedeutet, dass dieser Prozentsatz der geprüften Oberfläche nach wie vor mit dem geprüften Klebstoff bedeckt ist.

Für die Weiterverwendung des erfolgreich geprüften Bauteils, wird der Kohäsionsbruchanteil des Klebstoffes (also der verbliebene Klebstoffrest) an den geprüften Oberflächen vorzugsweise nicht entfernt. Die Antihaft- Materialschicht wird jedoch vollständig von der Bauteiloberfläche entfernt. Es ist anschließend möglich, erneut Klebstoff oder Dichtungsmaterial desselben Typs auf die ersten und zweiten Teilbereiche (jedoch ohne Antihaft-Materialschicht auf den ersten Teilbereichen) aufzubringen und auf diese Weise eine Klebstoffverbindung oder eine Dichtung zwischen zwei Bauteilen herzustellen.

Dieses erneute Aufbringen von Klebstoff auch auf Klebstoffreste erfolgt insbesondere nach einer Behandlung der Klebstoffreste mit einem haftvermittelnden Stoff (auch als Aktivator bezeichnet). Die verbliebenen Klebstoffreste an den geprüften Oberflächen können daher gemäß Vorgaben des Klebstoffherstellers mittels Aktivatoren "re-aktiviert" werden, so dass erneut Klebstoff oder Dichtungsmaterial desselben Typs auf die verbliebenen, "aktivierten" Klebstoffreste aufgetragen werden kann und auf diese Weise eine Montage des erfolgreich geprüften Bauteils ermöglicht werden kann. Diese Vorgehensweise ist bei einem Austausch von Fensterscheiben nach einem Unfall, eine gängige Methode. Nach dem Unfall wird die Klebverbindung der Fensterscheibe zum Beispiel zu einem Wagenkasten eines Schienenfahrzeugs mittels Schwingmessern aufgeschnitten, jedoch nicht von der Oberfläche entfernt. Eine neue Fensterscheibe wird schließlich über die re-aktivierten Klebstoffreste an den Wagenkasten angeklebt. Auch bei diesem bekannten Verfahren werden die Klebstoffreste nicht entfernt.

Bei Bauteiloberflächen, die eine Prüfung der Haftung lediglich auf der unmittelbaren Klebfläche ermöglichen, die eigentlich für die Montage des Bauteils vorgesehen ist (z.B. Keramiksiebdruckrand von Fensterscheiben), ist es zwingend notwendig Antihaft-Materialien zu verwenden, welche nachweislich rückstandsfrei von der Bauteiloberfläche abgezogen werden können. Wenn die Antihaft- Materialien vor oder nach Schritt a) aufgebracht werden, beginnt die Weiterverarbeitung der Bauteiloberfläche an den ersten Teilbereichen auch wieder bei Schritt a). Wenn die Antihaft- Materialien nachweislich rückstandsfrei ablösbar sind und die Antihaft- Materialien nach Schritt b) aufgetragen werden, werden die bereits mit Haftvermittlern vorbehandelten ersten Teilbereiche gemäß den Klebstoffherstellervorgaben für eine Weiterverwendung mit spezifischen Haftvermittlern (unter Umständen andere Stoffe als die Haftvermittler, die für die eigentliche Bauteiloberflächenbehandlung vorgesehen sind) "re- aktiviert". Nach "ReAktivierung" der ersten Teilbereiche und der Klebstoffreste ist eine Weiterverwendung schließlich ermöglicht.

Bei Bauteiloberflächen, die eine Prüfung der Haftung auf anderen Bereichen erlauben als die Bereiche, die für die Montage vorgesehen sind (z.B. undurchsichtiges Bauteil, wo die Prüfrückstände, wie Klebstoffreste, im Einbauzustand des Bauteils nicht sichtbar sind), wird es bevorzugt, auf diesen anderen Bereichen zu prüfen. Es ist anders als in dem oben beschriebenen Fall in diesem Fall nicht notwendig, das Antihaft-Material rückstandsfrei von dem Bauteil abzulösen. Vielmehr kann, optional wieder nach einer Vorbehandlung, erneut Klebstoff oder Dichtungsmaterial desselben Typs, jedoch auf andere Teilbereiche der Bauteiloberfläche als die ersten und zweiten Teilbereiche aufgebracht werden und auf diese Weise eine Klebstoffverbindung oder eine Dichtung zwischen zwei Bauteilen hergestellt werden.

Auch wenn der Klebstoff oder Dichtungsmaterial desselben Typs auf andere Teilbereiche der Bauteiloberfläche als zuvor bei dem Prüfvorgang aufgebracht wird, können durch die Prüfung zuverlässige Erkenntnisse über die Haftung des Klebstoffs an der Bauteiloberfläche gewonnen werden. Dies gilt insbesondere dann, wenn die gesamte Bauteiloberfläche, an der die Prüfung durchgeführt wird und auf die zur Herstellung einer Klebstoffverbindung oder einer Dichtung erneut Klebstoff oder Dichtungsmaterial aufgebracht wird, in gleicher Weise ausgestaltet ist.

Insbesondere kann die Antihaft-Materialschicht eine vor dem Aufbringen des Antihaft-Materials auf die Bauteiloberfläche bereits bestehende Materialschicht sein. Dies hat den Vorteil, dass die für das Aufbringen auf die Bauteiloberfläche benötigte Zeit verkürzt wird und über den gesamten ersten Teilbereich hinweg oder über die gesamten ersten Teilbereiche hinweg eine Antihaft-Materialschicht gleicher Art und Dicke aufgebracht werden kann.

Vorzugsweise weist die vorgefertigte Antihaft-Materialschicht zumindest einseitig einen Klebstofffilm auf. Es ist daher möglich, z.B. sogenannte Klebebänder zu verwenden. Insbesondere wird daher auf den zumindest einen ersten Teilbereich eine gleich große Fläche der Antihaft-Materialschicht aufgeklebt, wobei zum Aufkleben die an der Antihaft-Materialschicht vorhandene Klebstoffschicht verwendet wird. Im Ergebnis liegt diese Klebstoffschicht somit zwischen der eigentlichen Bauteiloberfläche und er durch das Antihaft-Material gebildeten Oberfläche, auf die der zu prüfende Klebstoff aufgebracht wird.

Insbesondere alternativ zur Verwendung von Klebebändern als Antihaft-Materialschicht kann eine vorgefertigte Antihaft-Materialschicht verwendet werden, die jedoch nicht vollflächig mit einer Klebstoffschicht versehen ist. Diese Antihaft-Materialschicht wird in bevorzugter Weise so auf die Bauteiloberfläche aufgebracht, dass sie sich streifenförmig quer zu der Längsrichtung des später aufgebrachten zu prüfenden Klebstoffs erstreckt. Dabei erstreckt sich die Antihaft-Materialschicht über eine Breite quer zu der Längsrichtung, die größer ist als die Breite des zu prüfenden aufgebrachten Klebstoffs. Insbesondere erstreckt sich die Antihaft-Materialschicht an beiden Seiten über den Bereich hinaus, auf den der zu prüfende Klebstoff aufgebracht wird. Ferner wird es bevorzugt, dass die Antihaft-Materialschicht in der Breitenrichtung seitlich neben den für den zu prüfenden Klebstoff vorgesehenen Bereichen auf der Bauteiloberfläche fixiert wird, z.B. mit einem Klebeband.

Eine derartige Antihaft-Materialschicht kann z.B. ein streifenförmiger Kabelbinder sein, der üblicherweise zum Zusammenbinden mehrerer elektrischer Kabel verwendet wird. Zum seitlichen Fixieren der Antihaft-Materialschicht kann z.B. Klebeband verwendet werden.

Alternativ können Streifen aus vorgefertigten Kunststoffplatten als Antihaft-Materialschicht verwendet werden. Auch aus Pappe oder Papier können Antihaft-Materialschicht-Stücke hergestellt werden, insbesondere Streifen. Dabei wird die Verwendung von fusselfreiem Papier oder fusselfreier Pappe bevorzugt. Auch aus Aluminiumfolie oder Schaumstoffplatten können Stücke der Antihaft-Materialschicht hergestellt, insbesondere zugeschnitten werden. Bei dem Schaumstoff handelt es sich z.B. um Melaminharzschaumstoff. Ferner ist vorzugsweise streifenförmiges PTFE (Polytetrafluorethylen) ein geeignetes Antihaft-Material.

Eine weitere Möglichkeit, die ersten und zweiten Teilbereiche für den Prüfvorgang herzustellen bzw. vorzubereiten, besteht in der Verwendung einer Schablone. Die Schablone kann aus dem Antihaft-Material gebildet sein und/oder dazu verwendet werden, Antihaft-Material auf die Bauteiloberfläche in durch die Schablone vorgegebenen Bereichen aufzubringen. Bevorzugt wird, dass die Schablone durch eine Materialschicht gebildet wird, die z.B. höchstens 1 mm dick ist. Die Schablone weist Aussparungen auf, die vorzugsweise streifenförmig sind und z. B. bei gleicher Streifenlänge in konstantem Abstand parallel zueinander verlaufen.

Wenn - wie bevorzugt - die Schablone aus Antihaft-Material gebildet ist, kann die Schablone auf die Bauteiloberfläche aufgelegt werden und/oder an der Bauteiloberfläche fixiert werden oder verfügt einseitig über einen Klebstofffilm, sodass die Schablone die Bauteiloberfläche mit Ausnahme der ausgesparten Bereiche abdeckt. Nun kann der zu prüfende Klebstoff auf die Schablone aufgebracht werden, sodass er sich durchgehend über von der Schablone abgedeckte erste Teilbereiche und über ausgesparte zweite Teilbereiche hinweg erstreckt.

Alternativ kann die Schablone auf die Bauteiloberfläche aufgebracht werden und/oder an der Bauteiloberfläche fixiert werden und/oder verfügt einseitig über einen Klebstofffilm und kann in den ausgesparten Bereichen der Schablone Antihaft-Material auf die Bauteiloberfläche aufgebracht werden. Anschließend wird die Schablone von der Bauteiloberfläche entfernt und verbleibt das in den ausgesparten Bereichen der Schablone auf die Bauteiloberfläche aufgebrachte Antihaft-Material an der Bauteiloberfläche. Anschließend kann der zu prüfende Klebstoff auf die Bauteiloberfläche aufgebracht werden, sodass er sich über zumindest einen mit Antihaft-Material versehenen Bereich hinweg erstreckt. Insbesondere kann die Schablone aus den zuvor genannten Antihaft-Materialien gebildet werden. Ferner ist es möglich, die parallel zueinander verlaufenden ausgesparten Bereiche der Schablone so breit auszugestalten, dass sie mindestens so breit wie der zu prüfende Klebstoff sind. Dabei können die ausgesparten Bereiche der Schablone an einander gegenüberliegenden Enden von dem Material der Schablone berandet werden. Vorzugsweise verfügt die Schablone einseitig über einen Klebstofffilm und kann somit an der Bauteiloberfläche fixiert werden. Alternativ werden die Bereiche der Schablone, die die ausgesparten Bereiche an den gegenüberliegenden Seiten beranden, an der Bauteiloberfläche fixiert, z.B. festgeklebt. Dabei können zusätzliche Klebebänder verwendet werden.

Als Ergebnis der Beurteilung der Haftung des Klebstoffs oder des Dichtungsmaterials kann die Entscheidung getroffen werden, dass das Bauteil für die Fertigung verwendet werden darf. Wenn die Haftung des Klebstoffs auf der Bauteiloberfläche jedoch nicht ausreicht, kann entschieden werden, dass das Bauteil nicht in der Fertigung verwendet werden darf.

Insbesondere auf diese Weise lässt sich die Verwendung zusätzlicher Muster, die von vornherein nicht für die Fertigung vorgesehen sind, vermeiden. Insbesondere wird eine Beschädigung durch scharfe spitze Werkzeuge, die gemäß dem vorbekannten Prüfverfahren verwendet werden, vermieden. Die für das Prüfverfahren verwendeten Bauteile unterscheiden sich daher nicht wie bei zusätzlichen Mustern von den in der Fertigung verwendeten Bauteilen. Kosten für zusätzliche Muster entfallen.

Ferner können Abstände zwischen den zweiten Teilbereichen der Bauteiloberfläche, auf die der Klebstoff unmittelbar aufgebracht wird, vorgegeben werden. Die Abstände müssen daher nicht wie bei der Verwendung eines scharfen spitzen Messers gemäß dem vorbekannten Prüfverfahren während der Prüfung gewählt werden. Durch Vorgabe der Größe der ersten Teilbereiche, die die zweiten Teilbereiche voneinander beabstanden, werden somit die Prüfbedingungen verschiedener Prüfvorgänge vergleichbar. Ferner ist die Wahrscheinlichkeit einer fehlerhaften Ausführung des bekannten Prüfverfahrens höher als die beschriebene Methode über erste- und zweite Teilbereiche. Bei dem bekannten Prüfverfahren besteht das Risiko, dass die Einschnitte nicht sachgemäß bis auf die Haftfläche erfolgen und dass ggf. der Einschnitt unter einem hohen Spannungszustand der Klebstoffraupe erfolgt, das zu lokalen Lackabplatzungen bei lackierten Proben oder Bauteilen führen kann.

Im Fall der oben bereits erwähnten Fensterscheiben ermöglicht das Prüfverfahren die Prüfung am Rand der Scheibe, wo sich eine undurchsichtige Farbschicht befindet und die Scheibe verklebt werden soll.

Ausführungsbeispiele der Erfindung werden nun unter Bezugnahme auf die beigefügte Zeichnung beschrieben. Die einzelnen Figuren der Zeichnung zeigen:
- Fig. 1: schematisch einen Querschnitt durch ein Bauteil mit einem auf die Bauteiloberfläche aufgebrachten Klebstoff, wobei erste Teilbereiche der Bauteiloberfläche mit einem Antihaft-Material versehen sind, sodass das Antihaft-Material zwischen dem Klebstoff und der Bauteiloberfläche angeordnet ist,
- Fig. 2: eine Darstellung wie in Fig. 1, wobei jedoch erkennbar ist, dass der Klebstoff außerhalb der ersten Teilbereiche in zweiten Teilbereichen der Bauteiloberfläche unmittelbar in Kontakt mit der Bauteiloberfläche ist,
- Fig. 3: schematisch eine Draufsicht auf die Anordnung von Fig. 1 und Fig. 2,
- Fig. 4: eine Draufsicht auf eine Schablone mit fünf parallelen streifenförmigen Aussparungen,
- Fig. 5 - 7: in einer Darstellung ähnlich der in Fig. 1 und 2, verschiedene Zustände während eines Prüfvorganges, bei dem der Klebstoff von der Bauteiloberfläche abgelöst wird.

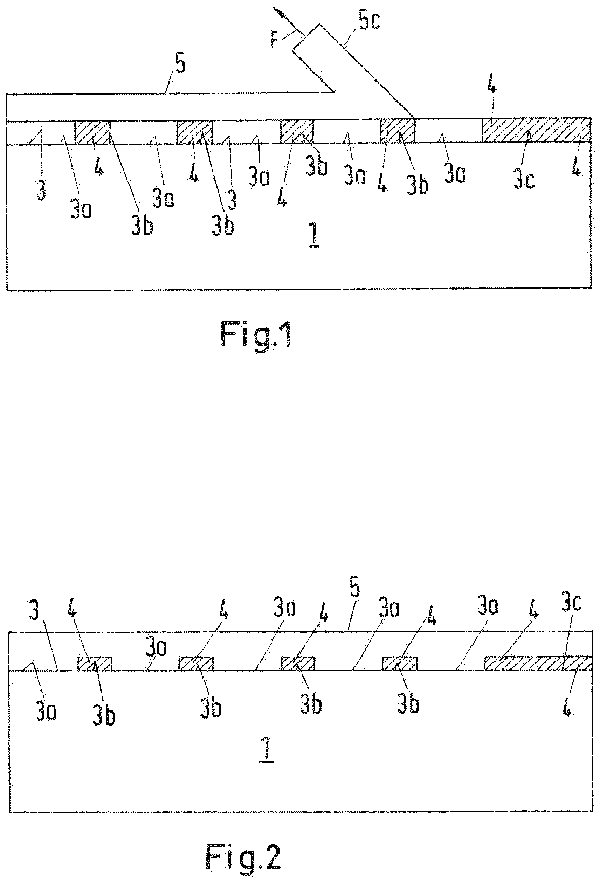

Fig. 1 zeigt nicht maßstäblich ein Bauteil 1, das an seiner Oberseite eine Bauteiloberfläche 3 bildet, die in dem Ausführungsbeispiel eben ist. Die Bauteiloberfläche 3 erstreckt sich in der Figur in horizontaler Richtung. Auf ersten Teilbereichen 3b, 3c der Bauteiloberfläche 3 ist eine Antihaft-Materialschicht 4 aufgebracht. Der besseren Erkennbarkeit wegen ist die Höhe bzw. Dicke der Antihaft-Materialschicht 4 übertrieben groß dargestellt. Auf den rechts in Fig. 1 dargestellten ersten Teilbereich 3c wird dasselbe Antihaft-Material aufgebracht wie auf die anderen ersten Teilbereichen 3b. Jedoch ist der erste Teilbereich 3c länger (in der von rechts nach links verlaufenden Längsrichtung) als die anderen ersten Teilbereiche 3b, um den Klebstoff mit einer geringen Schälkraft ablösen zu können. Z. B. wird der Klebstoff in dem ersten Teilbereich 3c auf einfache Weise mit einer Spitzzange abgelöst, um ihn über die (nach links in Fig. 1) folgenden ersten 3b und zweiten 3a Teilbereiche von der Bauteiloberfläche abschälen zu können. In der Verwendung einer Spitzzange unterscheidet sich das hier vorgestellte Verfahren nicht von dem bekannten Prüfverfahren.

Zwischen und neben den ersten Teilbereichen 3b, 3c befinden sich die zweiten Teilbereiche 3a, die nicht von der Antihaft-Materialschicht 4 bedeckt sind. In den ersten Teilbereichen 3b, 3c hat die Antihaft-Materialschicht 4 eine etwa konstante Dicke.

Auf die ersten Teilbereiche 3b, 3c und auf die zweiten Teilbereiche 3a ist Klebstoff 5 in Form einer Klebstoffraupe aufgebracht, dessen Haftung an der Bauteiloberfläche 3 geprüft werden soll. Die Klebstoffraupe ist in der bereits erwähnten Längsrichtung länglich geformt.

Fig. 2 zeigt die Anordnung aus Fig. 1, wobei jedoch die Dicke des Antihaft-Materials 4 weniger überhöht dargestellt ist und wobei erkennbar ist, dass der Klebstoff 5 sich zwischen der Antihaft-Materialschicht 4 bis zu den zweiten Teilbereichen 3a der Bauteiloberfläche 3 erstreckt.

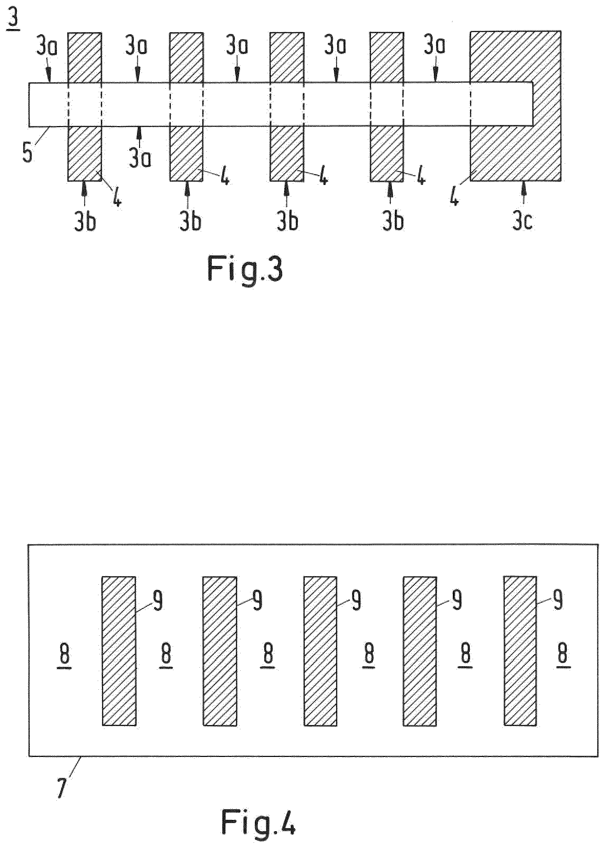

Die Draufsicht von Fig. 3 zeigt, dass sich die Antihaft-Materialschicht 4 streifenförmig quer zu einer in der Fig. 3 horizontal verlaufenden Längsachse des Klebstoffes 5 erstreckt. Die Breite der Streifen des Antihaft-Materials 4 in der vertikalen Richtung der Fig. 3 ist größer als die Breite des Klebstoffs 5. Die Antihaft-Materialschicht 4 erstreckt sich an beiden Seiten (oberhalb und unterhalb in der Darstellung der Fig. 3) des Klebstoffs 5. Die Antihaft-Materialschicht 4 deckt die ersten Teilbereiche 3b, 3c der Bauteiloberfläche 3 ab. Zwischen den ersten Teilbereichen 3b befinden sich die zweiten Teilbereiche 3a der Bauteiloberfläche 3, auf denen der Klebstoff 5 die Bauteiloberfläche unmittelbar, ohne zwischenliegende Antihaft-Materialschicht 4 kontaktiert.

In einer Ausführungsform des Prüfverfahrens wird zunächst das Antihaft-Material auf die ersten Teilbereiche 3b, 3c der Bauteiloberfläche 3 aufgebracht. Optional wird die Antihaft-Materialschicht 4 auf den ersten Teilbereichen 3b, 3c festgeklebt, z.B. über eine Klebstoffschicht an der Unterseite des Antihaft-Materials 4 oder mit Hilfe von nicht in Fig. 3 dargestellten Klebestreifen, die seitlich des zum Aufbringen des zu prüfenden Klebstoffes aufgebracht werden. Die Klebestreifen können insbesondere bei Bauteilen, bei denen die Haftung des Klebstoffes an der später für die Fertigung zu verwendenden Klebfläche geprüft werden muss (wie z.B. bei Fensterscheiben), über die Randzonen der Antihaft-Materialstreifen 4 hinweg, seitlich an der zu prüfenden Bauteiloberfläche 3 festgeklebt werden. Insbesondere kann auf diese Weise vermieden werden, dass Klebestreifen zur Befestigung des Antihaft-Materials 4 und/oder ein Klebstoff an der Unterseite des Antihaft-Materials 4, der der Befestigung des Antihaft-Materials 4 an der Bauteiloberfläche dient, auf die später für die Fertigung zu verwendende Klebfläche aufgeklebt wird/werden. Alternativ besteht jedoch auch die Möglichkeit dazu, wenn nachweislich eine rückstandsfreie Ablösung oder eine Möglichkeit der nachträglichen Reinigung (z.B. mit Lösemittelreinigern) dieser Stellen besteht.

Nach dem Aufbringen der Antihaft-Materialschicht 4 wird der Klebstoff 5 im Ausführungsbeispiel als Klebstoffraupe wie in der Draufsicht von Fig. 3 erkennbar aufgebracht. Die Klebstoffraupe hat z. B. einen halbrunden Querschnitt, d.h. ihr oberer Teil bildet die im Querschnitt halbrunde Form aus, während der Klebstoff unten auf dem z. B. jeweils annähernd ebenen Untergrund oberhalb der ersten Teilbereiche 3b, 3c und auf den zweiten Teilbereichen 3a aufliegt.

Nach dem Aushärten des Klebstoffs 5 wird eine Schälkraft F wie schematisch in Fig. 1 dargestellt auf den Klebstoff 5 ausgeübt, und zwar in einer Richtung, die mit der Längsrichtung einen Winkel von 130°bis 160° einschließt (Fig. 1). Die Längsrichtung ist dabei in Fig. 1 als die Richtung definiert, die sich längs der Erstreckung des Klebstoffs 5 von links nach rechts in der Fig. erstreckt. Da die Schälkraft F auf den Bereich rechts in Fig. 1 ausgeübt wird, kann die Schälkraft F den Klebstoff 5 nach links von der Bauteiloberfläche ablösen, sofern die Haftung des Klebstoffs dies erlaubt. Andernfalls führt die Erhöhung der Schälkraft F zu einem Kohäsionsbruch im Klebstoff. Der rechts in Fig. 1 dargestellte erste Teilbereich 3c mit darauf angeordneter Antihaft-Materialschicht 4 hat in dem in Fig. 1 dargestellten Zustand eine Ablösung des Endbereichs 5c des Klebstoffes 5 ermöglicht. Die Schälkraft F hat den Endbereich 5c außerdem vollständig von dem am weitesten rechts in Fig. 1 dargestellten zweiten Teilbereich 3a der Bauteiloberfläche 3 abgelöst. Die Haftung des Klebstoffes 5 ist daher ungenügend.

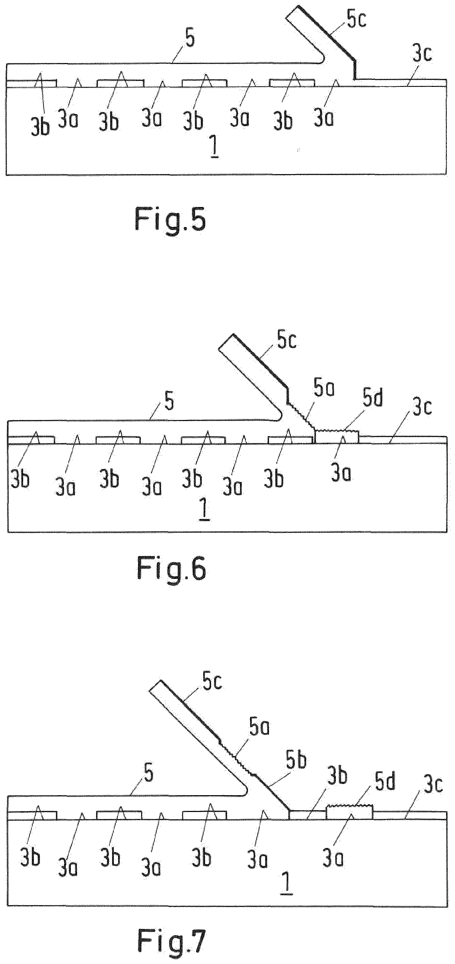

Es wird jedoch versucht, den Klebstoff 5 auch von den anderen verbleibenden Teilbereichen der Bauteiloberfläche 3 abzulösen, um die Haftung des Klebstoffes 5 an den anderen zweiten Teilbereichen 3a zu prüfen. Da die Haftwirkung des Klebstoffs 5 an der Antihaft-Materialschicht 4 und/oder die Haftwirkung des Antihaft-Materials 4 an der Bauteiloberfläche gering ist, wird der Klebstoff 5 im weiteren Verlauf des Prüfvorganges in dem (von rechts gesehen) zweiten ersten Teilbereich 3b von der Antihaft-Materialschicht 4 und/oder die Antihaft-Materialschicht 4 von der Bauteiloberfläche abgelöst und wirkt die Schälkraft F dann auf die Grenzfläche zwischen dem Klebstoff 5 und dem zweiten von insgesamt fünf dargestellten zweiten Teilbereichen 3a der Bauteiloberfläche 3. Dies ist nicht mehr in Fig. 1 dargestellt, ist aber zur Erläuterung eines anderen Verlaufs der Prüfung in Fig. 7 dargestellt. Es kann dann wieder beobachtet werden, ob Kohäsionsbrüche innerhalb des Klebstoffs 5 oder die Ablösung des Klebstoffs 5 von dem zweiten Teilbereich 3a der Bauteiloberfläche 3 überwiegen.

Wenn die in Fig. 1 dargestellte Klebstoffraupe vollständig (mit Ausnahme etwaig zurückbleibender Reste an der Bauteiloberfläche 3) von der Bauteiloberfläche 3 abgelöst worden ist, wird das Ergebnis der Ablösung unter Berücksichtigung von Kohäsionsbrüchen an den zweiten Teilbereichen 3a beurteilt. Ein Kohäsionsbruchanteil von mindestens 75% auf dem Gesamt-Anteil der zweiten Teilbereiche wird als gute Haftung des Klebstoffes an der Bauteiloberfläche gewertet. Dies entspricht auch der Bewertungsmethode des bekannten Prüfverfahrens.

Fig. 5 bis 7 zeigen ein anderes Beispiel für einen Verlauf der Prüfung ausgehend von der schematisch in Fig. 2 dargestellten Anfangssituation. Wie bereits anhand von Fig. 1 zuvor beschrieben, wird die Schälkraft F zunächst auf den Klebstoff 5 an dem ersten Teilbereich 3c der Bauteiloberfläche ausgeübt. Dies führt wieder zur vollständigen Ablösung des Endbereichs 5c des Klebstoffes 5 von dem ersten Teilbereich 3c, ohne dass es eines Einschnittes durch den Klebstoff 5 hindurch bis in die Bauteiloberfläche hinein bedarf. Der dadurch erreichte Zustand ist in Fig. 5 dargestellt.

Nun wird die Schälkraft F auf den angrenzenden zweiten Teilbereich 3a ausgeübt, an dem kein Antihaft-Material angeordnet ist. Von rechts gesehen in Fig. 5 bis 7 kann dieser Teilbereich 3a als erster der zweiten Teilbereiche 3a bezeichnet werden. Wie Fig. 6 zeigt wird der Klebstoff 5 zwar von diesem Teilbereich 3a abgelöst, jedoch bleibt über die gesamte Länge dieses Teilbereichs 3a ein Klebstoffrest 5d an der Bauteiloberfläche zurück. Dementsprechend weist die abgelöste Klebstoffraupe einen Kohäsionsbruch 5a auf. Die Haftung des Klebstoffes 5 an der Bauteiloberfläche in diesem Teilbereich 3a ist daher gut.

Nun kann der Prüfvorgang in einem entsprechenden Zustand wie in Fig. 1 gezeigt und oben beschrieben fortgesetzt werden. Aufgrund der reduzierten Haftung an dem von rechts gesehen zweiten der ersten Teilbereiche 3b, 3c findet eine vollständige Ablösung des Klebstoffes 5 von diesem Teilbereich 3b statt. Der dadurch erreichte Zustand ist in Fig. 7 dargestellt. Mit weiterem Fortschreiten des Prüfvorganges kann nun die Haftung des Klebstoffes 5 an dem (von rechts gesehen) zweiten der zweiten Teilbereiche 3a geprüft werden.

Fig. 4 zeigt eine Schablone 7 in Draufsicht. Die Schablone 7 besteht aus einer gleichmäßig dicken Schicht Material und weist fünf streifenförmige, parallel und in konstantem Abstand zueinander angeordnete Aussparungen 9 auf. Zwischen und neben den Aussparungen 9 befinden sich Materialbereiche 8 der Schablone. Die Schablone 7 kann wie auch andere Schablonen z.B. durch Ausstanzen der Aussparungen aus einem blattförmigen oder plattenförmigen Material hergestellt werden.

Wenn es sich bei dem Material der Schablone 7 um ein Antihaft-Material handelt, kann die Schablone derart auf einer Bauteiloberfläche angeordnet werden, dass ihr Material die Bauteiloberfläche in ersten Teilbereichen abdeckt, während die Aussparungen 9 zweite Teilbereiche der Bauteiloberfläche freilassen. Anschließend kann Klebstoff in der in Fig. 4 von links nach rechts verlaufenden Längsrichtung oder in umgekehrter Richtung auf die Schablone aufgebracht werden, sodass sich der Klebstoff über die Aussparungen 9 hinwegerstreckt. Die Anordnung des Klebstoffs in Fig. 4 kann dabei insbesondere in Bezug auf die Aussparungen 9 dieselbe sein, wie die Anordnung des Klebstoffs 5 in Fig. 3 in Bezug auf die Antihaft-Materialstreifen ist, d.h. es erstreckt sich eine Klebstoffraupe in einer Längsrichtung über die Aussparungen 9 bzw. die Antihaft-Materialstreifen hinweg.

Allerdings ist die Darstellung in Fig. 4 wie auch die Darstellung in den anderen Figuren schematisch zu verstehen. Die Abmessungen und die Anzahl der Antihaft-Materialbereiche und der Aussparungen können variieren. Insbesondere wird es bevorzugt, dass die Länge der Aussparungen 9 (gemessen in Längsrichtung von links nach rechts in Fig. 4 verlaufend) relativ zu den zwischen den Aussparungen 9 liegenden Materialbereichen 8 der Schablone 7 größer ist als in Fig. 4 dargestellt.

Anders als in Fig. 1 und Fig. 2 dargestellt, kann das Bauteil 1 an seiner Oberseite mehrere Schichten von verschiedenen Materialien aufweisen, wobei die oberste Materialschicht die Bauteiloberfläche bildet. Z.B. weist das Bauteil ein tragendes Substrat auf, auf dem sich zunächst von dem Substrat aus gesehen bis zur Bauteiloberfläche eine Grundierungsschicht befindet, darüber eine Zwischenlackschicht und wiederum darüber eine Decklackschicht. Optional kann auf der Decklackschicht noch eine Schicht eines haftvermittelnden Stoffes angeordnet sein, die die Haftung des Klebstoffs auf der Bauteiloberfläche verbessert.

## Patentansprüche

1. Verfahren zur Prüfung der Haftung von elastischen Klebstoffen oder elastischen Dichtungsmaterialien auf Oberflächen von Bauteilen, mit folgenden Schritten:
a) der Klebstoff (5) oder das Dichtungsmaterial wird auf einer Bauteiloberfläche (3) eines Bauteils aufgebracht,
b) es wird versucht, optional nach einem Aushärten des Klebstoffes (5) oder Dichtungsmaterials, durch Ausüben einer Schälkraft (F), den aufgebrachten Klebstoff (5) oder das aufgebrachte Dichtungsmaterial von der Bauteiloberfläche (3) abzulösen, und
c) anhand von durch das Ausüben der Schälkraft (F) bewirkten Brüchen in dem Klebstoff (5) oder dem Dichtungsmaterial einerseits und durch das Ausüben der Schälkraft (F) bewirkter Ablösung des Klebstoffes (5) oder des Dichtungsmaterials von der Bauteiloberfläche (3) andererseits wird die Haftung des Klebstoffes (5) oder des Dichtungsmaterials beurteilt,
wobei
vor und/oder während Schritt a) auf zumindest zwei erste Teilbereiche (3b) der Bauteiloberfläche (3) eine Antihaft-Materialschicht (4) aufgebracht wird, aufgrund der der Klebstoff (5) oder das Dichtungsmaterial an der Bauteiloberfläche (3) schlechter haftet und daher mit einer geringeren Schälkraft (F) von der Bauteiloberfläche (3) abgelöst werden kann als unmittelbar von der Bauteiloberfläche (3), und der Klebstoff (5) oder das Dichtungsmaterial in Schritt a) als durchgehender Materialbereich sowohl auf die zumindest zwei ersten Teilbereiche (3b) der Bauteiloberfläche (3), auf die die Antihaft-Materialschicht (4) aufgebracht wird und/oder wurde, und unmittelbar auf zumindest einen zweiten Teilbereich (3a) der Bauteiloberfläche (3) aufgebracht wird, sodass der Klebstoff (5) oder das Dichtungsmaterial unmittelbar mit der Bauteiloberfläche (3) in Kontakt ist,
wobei der Klebstoff (5) oder das Dichtungsmaterial derart auf die Bauteiloberfläche (3) aufgebracht wird, dass sich der Klebstoff (5) oder das Dichtungsmaterial in einer Längsrichtung seiner Erstreckung von einem der ersten Teilbereiche (3c) über einen der zweiten Teilbereiche (3a) zu einem anderen der ersten Teilbereiche (3b) erstreckt.

2. Verfahren nach dem vorhergehenden Anspruch, wobei der Klebstoff (5) oder das Dichtungsmaterial derart auf die Bauteiloberfläche (3) aufgebracht wird, dass sich der Klebstoff (5) oder das Dichtungsmaterial in der Längsrichtung über sich abwechselnd hintereinander angeordnete erste (3b) und zweite (3b) Teilbereiche der Bauteiloberfläche (3) erstreckt.

3. Verfahren nach dem vorhergehenden Anspruch, wobei die durch die Abmessungen der zweiten Teilbereiche (3a) in der Längsrichtung definierten Abstände der ersten Teilbereiche (3b) gleich groß sind.

4. Verfahren nach einem der vorhergehenden Ansprüche, wobei der zumindest eine zweite Teilbereich (3a) der Bauteiloberfläche (3) durch eine Schicht eines haftvermittelnden Stoffes gebildet wird, der die Haftung des Klebstoffes (5) oder des Dichtungsmaterials an dem Bauteil verbessert.

5. Verfahren nach einem der vorhergehenden Ansprüche, wobei die Antihaft-Materialschicht (4) vollständig und rückstandsfrei von der Bauteiloberfläche (3) entfernt wird und wobei anschließend erneut Klebstoff oder Dichtungsmaterial desselben Typs auf die ersten und zweiten Teilbereiche (3a) aufgebracht wird und auf diese Weise eine Klebstoffverbindung oder eine Dichtung zwischen zwei Bauteilen hergestellt wird.

6. Verfahren nach einem der vorhergehenden Ansprüche, wobei erneut Klebstoff oder Dichtungsmaterial desselben Typs auf andere Teilbereiche der Bauteiloberfläche (3) als die ersten und zweiten Teilbereiche (3a) aufgebracht wird und auf diese Weise eine Klebstoffverbindung oder eine Dichtung zwischen zwei Bauteilen hergestellt wird.

7. Anordnung zur Prüfung der Haftung von elastischen Klebstoffen oder elastischen Dichtungsmaterialien auf Oberflächen von Bauteilen, mit Klebstoff oder Dichtungsmaterial, der/das auf eine Bauteiloberfläche (3) eines Bauteils aufgebracht ist,
aufweisend
eine Antihaft-Materialschicht (4), aufgrund der der Klebstoff (5) oder das Dichtungsmaterial an der Bauteiloberfläche (3) schlechter haftet und daher mit einer geringeren Schälkraft (F) von der Bauteiloberfläche (3) abgelöst werden kann als unmittelbar von der Bauteiloberfläche (3), wobei der Klebstoff (5) oder das Dichtungsmaterial als durchgehender Materialbereich sowohl auf zumindest einem ersten Teilbereich (3b) der Bauteiloberfläche (3) mit zwischen dem Klebstoff (5) und der Bauteiloberfläche (3) liegender Antihaft-Materialschicht (4) als auch unmittelbar auf zumindest einem zweiten Teilbereich (3a) der Bauteiloberfläche (3) ohne Antihaft-Materialschicht (4) aufgebracht ist, sodass der Klebstoff (5) oder das Dichtungsmaterial unmittelbar mit der Bauteiloberfläche (3) in Kontakt ist, wobei der Klebstoff (5) oder das Dichtungsmaterial derart auf die Bauteiloberfläche (3) aufgebracht ist, dass sich der Klebstoff (5) oder das Dichtungsmaterial in einer Längsrichtung seiner Erstreckung zumindest von einem der ersten Teilbereiche (3c) über einen der zweiten Teilbereiche (3a) zu einem anderen der ersten Teilbereiche (3b) erstreckt.

8. Anordnung nach Anspruch 7, wobei der Klebstoff (5) oder das Dichtungsmaterial derart auf die Bauteiloberfläche (3) aufgebracht ist, dass sich der Klebstoff (5) oder das Dichtungsmaterial in der Längsrichtung über sich abwechselnd hintereinander angeordnete erste und zweite Teilbereiche (3a) der Bauteiloberfläche (3) erstreckt.

9. Anordnung nach Anspruch 8, wobei die durch die Abmessungen der zweiten Teilbereiche (3a) in der Längsrichtung definierten Abstände der ersten Teilbereiche (3b) gleich groß sind.

## Claims

1. A method for testing the adhesion of elastic adhesives or elastic sealing materials on surfaces of components, comprising the following steps:
a) applying the adhesive (5) or the sealing material to a component surface (3) of a component;
b) attempting, by exerting a peeling force (F), to detach the applied adhesive (5) or the applied sealing material from the component surface (3), optionally after curing of the adhesive (5) or the sealing material; and
c) assessing the adhesion of the adhesive (5) or of the sealing material based on fractures caused in the adhesive (5) or the sealing material by the exertion of the peeling force (F) on the one hand, and based on the detachment of the adhesive (5) or of the sealing material from the component surface (3) by the exertion of the peeling force (F) on the other hand,
wherein
prior to and/or during step a), an anti-adhesion material layer (4) is applied to at least two first sub-regions (3b) of the component surface (3), as a result of which the adhesive (5) or the sealing material exhibits poorer adhesion to the component surface (3) and therefore can be detached from the component surface (3) using a lower peeling force (F) than directly from the component surface (3), and the adhesive (5) or the sealing material in step a) is applied as a continuous material region both to the at least two first sub-regions (3b) of the component surface (3) to which the anti-adhesion material layer (4) is and/or was applied, and directly to at least one second sub-region (3a) of the component surface (3), so that the adhesive (5) or the sealing material is in direct contact with the component surface (3),
wherein the adhesive (5) or the sealing material is applied to the component surface (3) in such a way that the adhesive (5) or the sealing material extends in a longitudinal direction of the extension thereof from one of the first sub-regions (3c) over one of the second sub-regions (3a) to another of the first sub-regions (3b).

2. The method according to the preceding claim, wherein the adhesive (5) or the sealing material is applied to the component surface (3) in such a way that the adhesive (5) or the sealing material extends, in the longitudinal direction, over first (3b) and second (3b) sub-regions of the component surface (3) disposed alternately in succession.

3. The method according to the preceding claim, wherein the distances of the first sub-regions (3b) defined by the dimensions of the second sub-regions (3a) in the longitudinal direction are equal in size.

4. The method according to any one of the preceding claims, wherein the at least one second sub-region (3a) of the component surface (3) is formed by a layer of an adhesion-promoting substance which improves the adhesion of the adhesive (5) or of the sealing material to the component.

5. The method according to any one of the preceding claims, wherein the anti-adhesion material layer (4) is removed from the component surface (3) completely and without residue, and thereafter adhesive or sealing material of the same type is again applied to the first and second sub-regions (3a), and in this way an adhesive bond or a seal between two components is established.

6. The method according to any one of the preceding claims, wherein adhesive or sealing material of the same type is again applied to sub-regions of the component surface (3) other than the first and second sub-regions (3a), and in this way an adhesive bond or a seal between two components is established.

7. A system for testing the adhesion of elastic adhesives or elastic sealing materials to surfaces of components, with adhesive or sealing material applied to a component surface (3) of a component,
comprising
an anti-adhesion material layer (4), as a result of which the adhesive (5) or the sealing material exhibits poorer adhesion to the component surface (3) and therefore can be detached from the component surface (3) using a lower peeling force (F) than directly from the component surface (3), wherein the adhesive (5) or the sealing material is applied as a continuous material region both to at least one first sub-region (3b) of the component surface (3), with the anti-adhesion material layer (4) disposed between the adhesive (5) and the component surface (3), and also directly to at least one second sub-region (3a) of the component surface (3) without anti-adhesion material layer (4), so that the adhesive (5) or the sealing material is in direct contact with the component surface (3),
wherein the adhesive (5) or the sealing material is applied to the component surface (3) in such a way that the adhesive (5) or the sealing material extends in a longitudinal direction of the extension thereof from at least one of the first sub-regions (3c) over one of the second sub-regions (3a) to another of the first sub-regions (3b).

8. The system according to claim 7, wherein the adhesive (5) or the sealing material is applied to the component surface (3) in such a way that the adhesive (5) or the sealing material extends, in the longitudinal direction, over first and second sub-regions (3a) of the component surface (3) disposed alternately in succession.

9. The system according claim 8, wherein the distances of the first sub-regions (3b) defined by the dimensions of the second sub-regions (3a) in the longitudinal direction are equal in size.

## Revendications

1. Procédé de contrôle de l'adhérence d'adhésifs élastiques ou de matériaux d'étanchéité élastiques sur des surfaces de pièces, comprenant les étapes suivantes :
a) l'adhésif (5) ou le matériau d'étanchéité est appliqué sur une surface de pièce (3) d'une pièce,
b) on essaye de décoller de la surface de pièce (3) l'adhésif (5) appliqué ou le matériau d'étanchéité appliqué par l'exercice d'une force de pelage (F), éventuellement après un durcissement de l'adhésif (5) ou du matériau d'étanchéité, et
c) l'adhérence de l'adhésif (5) ou du matériau d'étanchéité est évaluée sur la base d'une part de fractures dans l'adhésif (5) ou le matériau d'étanchéité provoquées par l'exercice de la force de pelage (F) et d'autre part du décollement de l'adhésif (5) ou du matériau d'étanchéité de la surface de pièce (3) provoqué par l'exercice de la force de pelage (F),
dans lequel
avant et/ou pendant l'étape a), une couche de matériau antiadhésif (4) est appliquée sur au moins deux premières zones partielles (3b) de la surface de pièce (3), en raison de laquelle l'adhésif (5) ou le matériau d'étanchéité adhère moins bien à la surface de pièce (3) et peut donc être décollé de la surface de pièce (3) avec une force de pelage (F) moindre que directement de la surface de pièce (3), et l'adhésif (5) ou le matériau d'étanchéité est appliqué à l'étape a) sous la forme d'une zone de matériau continue aussi bien sur les au moins deux premières zones partielles (3b) de la surface de pièce (3), sur laquelle la couche de matériau antiadhésif (4) est et/ou a été appliquée, et directement sur au moins une deuxième zone partielle (3a) de la surface de pièce (3), de sorte que l'adhésif (5) ou le matériau d'étanchéité soit directement en contact avec la surface de pièce (3),
dans lequel l'adhésif (5) ou le matériau d'étanchéité est appliqué sur la surface de pièce (3) de telle sorte que l'adhésif (5) ou le matériau d'étanchéité s'étend dans une direction longitudinale de son étendue d'une des premières zones partielles (3c) vers une autre des premières zones partielles (3b) en passant par une des deuxièmes zones partielles (3a).

2. Procédé selon la revendication précédente, dans lequel l'adhésif (5) ou le matériau d'étanchéité est appliqué sur la surface de pièce (3) de telle sorte que l'adhésif (5) ou le matériau d'étanchéité s'étend dans la direction longitudinale sur des premières (3b) et deuxièmes (3b) zones partielles de la surface de pièce (3) disposées en alternance les unes derrière les autres.

3. Procédé selon la revendication précédente, dans lequel les écarts des premières zones partielles (3b) définis par les dimensions des deuxièmes zones partielles (3a) dans la direction longitudinale sont identiques.

4. Procédé selon l'une quelconque des revendications précédentes, dans lequel l'au moins une deuxième zone partielle (3a) de la surface de pièce (3) est formée par une couche d'une substance promotrice d'adhésion, qui améliore l'adhérence de l'adhésif (5) ou du matériau d'étanchéité sur la pièce.

5. Procédé selon l'une quelconque des revendications précédentes, dans lequel la couche de matériau antiadhésif (4) est retirée de la surface de pièce (3) entièrement et sans laisser de résidus et dans lequel un adhésif ou un matériau d'étanchéité du même type est ensuite à nouveau appliqué sur les premières et deuxièmes zones partielles (3a) et de cette manière une liaison adhésive ou un joint entre deux pièces est obtenu.

6. Procédé selon l'une quelconque des revendications précédentes, dans lequel un adhésif ou un matériau d'étanchéité du même type est à nouveau appliqué sur d'autres zones partielles de la surface de pièce (3) que les premières et deuxièmes zones partielles (3a) et de cette manière une liaison adhésive ou un joint entre deux pièces est obtenu.

7. Ensemble pour le contrôle de l'adhérence d'adhésifs élastiques ou de matériaux d'étanchéité élastiques sur des surfaces de pièces, avec un adhésif ou un matériau d'étanchéité qui est appliqué sur une surface de pièce (3) d'une pièce,
présentant
une couche de matériau antiadhésif (4), en raison de laquelle l'adhésif (5) ou le matériau d'étanchéité adhère moins bien à la surface de pièce (3) et peut donc être décollé de la surface de pièce (3) avec une force de pelage (F) moindre que directement de la surface de pièce (3), dans lequel l'adhésif (5) ou le matériau d'étanchéité est appliqué sous la forme d'une zone de matériau continue aussi bien sur au moins une première zone partielle (3b) de la surface de pièce (3), avec une couche de matériau antiadhésif (4) située entre l'adhésif (5) et la surface de pièce (3), que directement sur au moins une deuxième zone partielle (3a) de la surface de pièce (3) sans couche de matériau antiadhésif (4), de sorte que l'adhésif (5) ou le matériau d'étanchéité soit directement en contact avec la surface de pièce (3),
dans lequel l'adhésif (5) ou le matériau d'étanchéité est appliqué sur la surface de pièce (3) de telle sorte que l'adhésif (5) ou le matériau d'étanchéité s'étend dans une direction longitudinale de son étendue d'au moins une des premières zones partielles (3c) vers une autre des premières zones partielles (3b) en passant par une des deuxièmes zones partielles (3a).

8. Ensemble selon la revendication 7, dans lequel l'adhésif (5) ou le matériau d'étanchéité est appliqué sur la surface de pièce (3) de telle sorte que l'adhésif (5) ou le matériau d'étanchéité s'étend dans la direction longitudinale sur des premières et deuxièmes zones partielles (3a) de la surface de pièce (3) disposées en alternance les unes derrière les autres.

9. Ensemble selon la revendication 8, dans lequel les écarts des premières zones partielles (3b) définis par les dimensions des deuxièmes zones partielles (3a) dans la direction longitudinale sont identiques.
